# EUROPEAN PATENT APPLICATION

(11) **EP 0 610 575 A2**
(43) Date of publication of application: **17.08.1994**
(21) Application number: 93120021.6
(22) Date of filing: 11.12.1993
(51) Int. Cl.: A61F 2/28, A61B 17/56

(54) **Removable stump pin for endosteal implants**

(30) Priority: 11.02.1993 IT MI930250
(71) Applicant: Vrespa, Giuseppe, I-20025 Legnano (Milan) (IT)
(72) Inventor: Vrespa, Giuseppe, I-20025 Legnano (Milan) (IT)
(74) Representative: Giambrocono, Alfonso, Dr. Ing.

(57) **Abstract**

The stump pin device (14) for endosteal implants comprises a body (16) which can be expanded after insertion into the relative cavity (12) in the endosteal device (10). The expandability of the expandable body (16) is reversible so as to make the stump pin device removable.

## Description

This invention relates to a stump pin device for endosteal implants, ie a device enabling a prosthesis to be fixed to any endosteal device, which may be for example an endosteal screw or a fixing foot.

As is known to the expert of the art, the law in many countries (such as the United States of America) requires that the stump pin be removable from an endosteal implant or fixture. To achieve this removability, all currently available devices comprise a threaded stump pin to be screwed into a female thread present in the endosteal device, and hence unscrewable to achieve the required removability of the stump pin.

The main drawback of such known stump pins is that they are subject to accidental unscrewing. Recent research at an international level (T. Jemt, Quintessence International, 2/1992) on single implants shows that in 26% of the cases the stump pin suffered accidental unscrewing.

Prismatic stump pins are also known, these being of considerable advantage both because they allow high positioning accuracy in the implant and because they can be formed of the most suitable metal for the particular prosthesis by casting, using a conventional calcinable substance (disposable wax). Notwithstanding said merits, prismatic stump pins have been abandoned because as the stump pin has to be cemented to fix it to the implant it is not removable.

The object of the present invention is to provide a stump pin which although offering the advantages of prismatic stump pins is removable.

This object is attained by the stump pin device of the present invention, which is characterised by comprising an expandable body insertable into the relative cavity in the endosteal device, the expandability of said body being reversible.

Preferably said expandable body of the stump pin device is of overall tubular shape and is arranged to receive a screw element, the expansion of the tubular body being achieved by screwing the screw element.

In this manner a stump pin resembling an expansion plug is formed, such that on screwing the screw element the stump pin becomes locked within the endosteal device, whereas on unscrewing the screw element the stump pin is released and can be extracted, so achieving the required removability of the stump pin.

Said expandable body of the stump pin device of the present invention can be prismatic as in conventional prismatic stump pins, so presenting all the advantages thereof plus removability.

Alternatively the expandable body can be provided with an external thread as in the case of currently used screwable and unscrewable stump pins, or where the expandable body forms part of a transepithelial fixing screw or of other analogous devices.

However, because of the expandability characteristic, which enables the stump pin device to be locked permanently to the endosteal device, it cannot unscrew accidentally even though the expandable body is of the screwable type.

Preferably in the case of the stump pin device with a tubular body expandable by inserting a screw element into it, the hole through the tubular body has a flared end portion, the tubular body comprising at least two radial slots extending through a certain distance from that end of the tubular body at which said flared portion is located, the screw element comprising a screw of length greater than the hole through the body and a wedge-shaped nut screwable onto the screw and forcibly insertable into the flared portion of the tubular body.

Consequently by inserting the screw through the hole in the expandable body such that the end of the screw emerges from that end of said body at which said flaring is located, then screwing onto that end of the screw projecting from the body the wedge-shaped nut until it makes contact with the relative flaring, then placing the thus complete stump pin device into the relative seat provided in the endosteal device and finally screwing the screw, that portion of the body comprising said radial slots expands to hence lock the stump pin of the endosteal device in position.

The invention will be more apparent from the following description of one embodiment thereof given by way of example only.

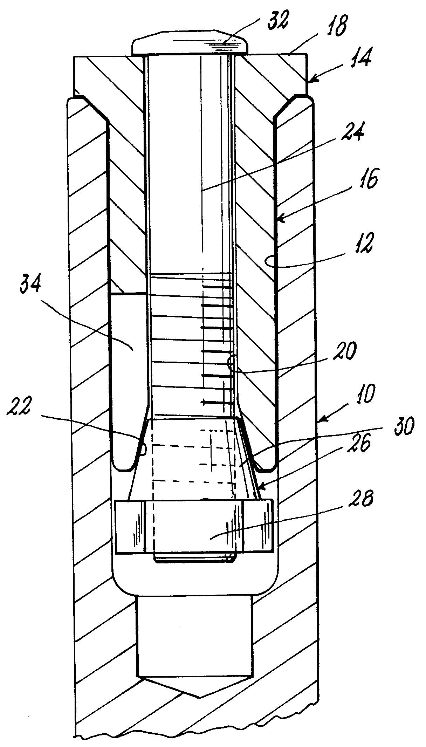

In this description reference is made to the accompanying drawing, in which the single figure represents a cross-section through the stump pin device already inserted in the relative endosteal device.

This figure shows a generic endosteal device, indicated overall by the reference numeral 10, of which only the upper part which receives the stump pin device 14 is visible.

The endosteal device 10 can for example be an endosteal screw, a fusion fixing foot or the head of a hip prosthesis. In each case the endosteal device 10 comprises a hole 12 into which the stump pin device is inserted. In the specific case represented in the figure, the stump pin 14 is shown already inserted into the hole 12 of the endosteal device 10, and consists of a tubular body 16 which is lowerly expandable because of the presence of three radial slots (of which only one, 34, is visible in the figure).

The tubular body 16 has an upper flange 18 which rests on the upper edge of the endosteal device 10 to facilitate positioning of the stump pin.

As can be seen from the figure, the hole 20 in the tubular body 16 has a flared portion 22 at its lower mouth. Through the hole 20 there is already inserted a screw 24 of length such as to project a certain distance beyond the body 16 so that a wedge-shaped nut 26 can be screwed thereon. This nut consists of a lower part 28 in the form of a conventional screw nut, and an upper frusto-conical part 30 coaxial with and insertable in the manner of a wedge into the flaring 22 of the body 16. In the figure the nut 26 is shown in the position in which it has engaged the body 16, As will be apparent observing the figure, it is not necessary for the whole of the screw shank to be threaded, it being sufficient to thread only that portion on which the wedge-shaped nut 26 is screwed. The screw 24 has a normal head 32 comprising a conventional recess for receiving the tip of a tool (screwdriver or the like) for screwing and unscrewing the screw.

As stated, the tubular body 16 can be of polygonal cross-section (for example hexagonal) to provide a true stump pin of prismatic (hexagonal) type, or alternatively be of circular cross-section with its outer lateral surface threaded. The cavity in the endosteal device must then be of hexagonal cross-section or of female thread form respectively, to receive the relative expandable tubular body. In all cases the stump pin provided is removable by unscrewing the screw 24. The device is also conceived to allow the stump pin to be used in any situation of misalignment between the prosthetic crown and the direction of the implant in the bone.

The tubular body 16 can be conveniently formed of a suitable metal, or of a convenient calcinable material followed by metal casting.

## Claims

1. A stump pin device for endosteal implants, characterised by comprising an expandable body insertable into the relative cavity in the endosteal device, the expandability of said body being reversible.

2. A device as claimed in claim 1, wherein the expandable body of the stump pin device is of overall tubular shape and is arranged to receive a screw element, the expansion of the tubular body being achieved by screwing the screw element.

3. A device as claimed in claim 2, wherein the hole through the tubular body has a flared end portion, the tubular body comprising at least two radial slots extending through a certain distance from that end of the tubular body at which said flared portion is located, the screw element comprising a screw of length greater than the hole through the tubular body and a wedge-shaped nut screwable onto the screw and forcibly insertable into the flared portion of the tubular body.

4. A device as claimed in claim 1, wherein the cross-section through the expandable body is polygonal, the expandable body being conjugate with the relative cavity, also polygonal, in the endosteal device.

5. A device as claimed in claim 1, wherein the cross-section through the expandable body is circular, the outer lateral surface of said expandable body being threaded and screwable into the relative cavity in the endosteal device.

6. A device as claimed in claim 1, wherein the expandable body is formed of calcinable material and then cast in a suitable metal.
